(19) Europäisches Patentamt
European Patent Office
Office européen des brevets

(11) Publication number: 0 055 145
B1

(12) **EUROPEAN PATENT SPECIFICATION**

(45) Date of publication of patent specification: **05.12.84**

(51) Int. Cl.$^3$: **C 07 D 317/58,** A 61 K 31/36

(21) Application number: **81306186.8**

(22) Date of filing: **22.12.81**

(54) **4-(Aminomethyl)cyclohexane-1-carboxylic acid derivatives.**

(30) Priority: **22.12.80 JP 181707/80**

(43) Date of publication of application:
**30.06.82 Bulletin 82/26**

(45) Publication of the grant of the patent:
**05.12.84 Bulletin 84/49**

(84) Designated Contracting States:
**BE CH DE FR GB IT LI NL**

(56) References cited:
**GB-A-1 544 969**
**US-A-3 894 051**

(73) Proprietor: **KUREHA KAGAKU KOGYO KABUSHIKI KAISHA**
**9-11 Horidome-cho 1-chome Nihonbashi Chuoku**
**Tokyo (JP)**

(72) Inventor: **Takita, Hitoshi**
**1-8 Toyotama-kita Nerima-ku**
**Tokyo (JP)**
Inventor: **Noda, Sakuo**
**3-26-1 Hyakunin-cho Shinjuku-ku**
**Tokyo (JP)**
Inventor: **Mukaida, Yutaka**
**No. 21 Daiichi-Kurehasoh 3-10-13 Nerima**
**Nerima-ku Tokyo (JP)**
Inventor: **Kobayashi, Hidetoshi**
**2-21-2 Zenpukuji Suginami-ku**
**Tokyo (JP)**

(74) Representative: **Myerscough, Philip Boyd et al**
**J.A.Kemp & Co. 14, South Square Gray's Inn**
**London, WC1R 5EU (GB)**

**EP 0 055 145 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to 4-(aminomethyl)cyclohexane-1-carboxylic acid derivatives, to their preparation and to pharmaceutical compositions containing them.

The present invention provides 4-[N-(3′,4′-methylenedioxybenzylidene)aminomethyl]cyclohexane-1-carboxylic acid, which is a compound represented by the formula (I):

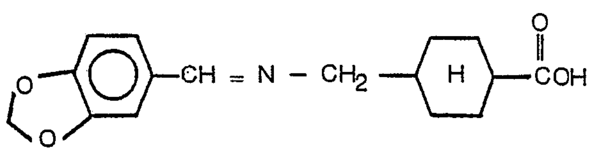

(I)

or a $C_1$—$C_4$ alkyl ester thereof or a pharmaceutically acceptable salt thereof, such as the sodium salt or ammonium salt. The cyclohexane ring may be present in the trans- or cis-forms or in a mixture thereof. The compounds of the invention exhibit a low toxicity in mammals and can be used in the treatment of inflammation, thrombosis, hypertension, asthma, cancer, rheumatism or systemic lupus erythematosus.

The compounds of the invention are preferably prepared by reacting 3,4-methylenedioxybenzaldehyde, which is a compound of formula (II):

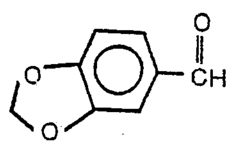

(II)

with a 4-aminomethyl-cyclohexane-1-carboxylic acid or an ester thereof represented by the formula (III):

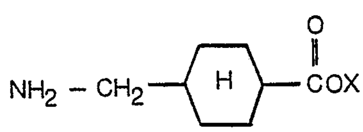

(III),

wherein the cyclohexane ring may be in the trans- or cis- form or in a mixture of trans- and cis-forms and X represents hydrogen or an alkyl group of from 1 to 4 carbon atoms, and optionally converting the resulting compound into a pharmaceutically acceptable salt.

The reaction between the compound of the formula (II) and the compound of the formula (III) is a dehydration-condensation reaction and is preferably carried out in an organic solvent at not higher than 150°C, preferably at a temperature of from 0 to 120°C under an atmosphere of an inert gas. At higher than 150°C, the yield of the desired product is reduced because of various side reactions. Any organic solvent may be used provided it does not participate in the reaction. A lower alcohol such as methanol or ethanol; benzene; toluene; dimethylformamide; acetonitrile or the like can be employed.

Since the reaction takes place with dehydration, the reaction is preferably carried out in the presence of a dehydrating agent or while removing water formed by the reaction under the reflux of the solvent. An anhydrous lower alcohol such as anhydrous methanol or ethanol can be used as the solvent and at the same time as the dehydrating agent.

The compound of the invention (acid- or ester form) can be isolated by treating the resulting reaction mixture by a known method. The salt form can be prepared by the conventional neutralization method using a base such as hydroxide, carbonate or bicarbonate of an alkali or alkaline earth metal, for example, sodium, potassium, calcium or magnesium, or ammonia or a primary-, secondary- or tertiary amine or a quaternary ammonium salt. For example, a sodium salt can be obtained by neutralizing the compound of the invention (an acid form) obtained by the above-mentioned reaction with an alcoholic- or aqueous solution of sodium hydroxide under an inert gas atmosphere at lower than 100°C, usually at 0 to 50°C.

The compounds of the invention show an inhibitory effect on platelet aggregation and/or polymorphonuclear leukocyte migration, and a low acute toxicity, as will be shown in Example 5 below. Accordingly, the compounds of the invention are useful as a remedy for various diseases, such as inflammation, thrombosis, hypertension, asthma or cancer, and especially for chronic diseases such as rheumatism or systemic lupus erythematosus (SLE). When used for pharmaceutical purposes, the salt or ester must be pharmaceutically acceptable.

The present invention further provides a pharmaceutical composition which comprises as active ingredient a compound of formula (I) or a $C_1$—$C_4$ alkyl ester thereof or a pharmaceutically acceptable salt thereof, together with a pharmaceutically acceptable carrier or diluent.

The compound of the invention can be administered perorally, rectally or by injection in various dosage forms as a composition together with a pharmaceutically acceptable carrier or diluent. The composition can be in unit dosage form, containing a therapeutically effective amount of a compound of the invention. Conventional pharmaceutical adjuvants may be present. Two or more of the compounds of the invention and/or other pharmaceutically active materials may be present in the pharmaceutical composition.

The composition may be in the form of a tablet, sublingual tablet, powder, capsule, troch, aqueous or oily solution, suspension, emulsion, syrup, aqueous or oily injection. Examples of the carrier or diluent mentioned above include water, gelatin, lactose, starch, pectin, magnesium stearate, stearic acid, talc, vegetable oil, gum arabic, polyalkylene glycol, vaseline, sorbitan trioleate, polyoxyethylene-sorbitan mono-oleate, alkylphenol, aliphatic alcohols and polyvinyl pyrrolidone. In the composition, if necessary, an edulcorant, flavouring agent, tinctorial agent, preservative, salt for osmoregulation or buffer, that is a conventional pharmaceutical adjuvant, may be present.

The content of a compound of the invention in the pharmaceutical composition may be varied, for example, from 0.01% to less than 100% by weight of the composition preferably 0.05%—80% by weight of the composition.

The pharmaceutical composition of the invention can be administered to a human or animal parenterally, for example, rectally, by injection (hypodermic, intramuscular or intravenous, or drip), also preferably perorally (for example sublingual etc.).

A dose of the pharmaceutical composition of the invention is typically 0.1 to 500 mg, preferably 0.5 to 200 mg, per day per kilogram of body weight in the case of peroral administration to a human, and 0.01 to 200 mg, preferably 0.1 to 100 mg, in the case of parenteral administration. The dose can be administered in from one to four portions a day. However, the dose depends on age, individuality, condition of a disease, etc. of the human or animal patient, and may therefore be outside the above-mentioned range.

The method for preparation, properties and pharmacological effects of compounds of the invention are illustrated in the following Examples. Reference is made in the Examples to the accompanying drawings in which:

Figure 1 shows an infrared absorption spectrum of sodium 4-[N-(3′,4′-methylenedioxybenzylidene)aminomethyl]cyclohexane-1-carboxylate taken using a KBr tablet;

Figure 2 shows an infrared absorption spectrum of methyl 4-[N-(3′,4′-methylenedioxybenzylidene)aminomethyl]cyclohexane-1-carboxylate taken using a KBr tablet; and

Figure 3 shows an infrared absorption spectrum of ethyl 4-[N-(3′,4′-methylenedioxybenzylidene)aminomethyl]cyclohexane-1-carboxylate taken using a KBr tablet.

## Example 1

Preparation of 4-[N-(3′,4′-methylenedioxybenzylidene)aminomethyl]cyclohexane-1-carboxylic acid:

1.91 Grams (12.72 mmol) of 3,4-methylenedioxybenzaldehyde was dissolved in 30 ml of dehydrated and purified methanol . The resulting solution was added dropwise into 2.00 g (12.72 mmol) of trans-4-aminomethyl-cyclohexane-1-carboxylic acid under an atmosphere of nitrogen. The resulting mixed solution was refluxed with stirring for 3 hours.

Small amounts of insoluble materials were removed by filtration. The filtrate was concentrated and left overnight at room temperature. Educing crystals were collected by filtration, washed with methanol and vacuum dried. 1.22 Grams of 4-[N-(3′,4′-methylenedioxybenzylidene)aminomethyl]-cyclohexane-1-carboxylic acid melting at 146 to 147°C (according to hot plate method) was obtained. The yield was 33.2%.

The characteristics of this compound of the invention thus obtained were as follows:

(1) Elementary analytical follows:

| | | | |
|---|---|---|---|
| Found (%) | C: 66.30, | H: 6.70, | N: 4.90 |
| Calcd.(%) as $C_{16}H_{19}O_4N$, | C: 66.42, | H: 6.62, | N: 4.84 |

(2) NMR: $\delta$ = 1.03~1.90 (9H, m), 1.99~2.13(1H), 3.36(2H, d), 6.06(2H, s), 6.95(1H, d), 7.19(1H, d), 7.28(1H, s) and 8.15 (1H, s).

Example 2

Preparation of sodium 4 - [N - (3',4' - methylenedioxybenzylidene) - aminomethyl]cyclohexane - 1-carboxylate:

2.893 Grams (10.0 m mol) of 4-[N-(3',4'-methylenedioxybenzylidene)aminomethyl]cyclohexane-1-carboxylic acid obtained in Example 1 was added under an atmosphere of nitrogen into 100 ml (10.0 m mol) of 0.1 N aqueous solution of sodium hydroxide. The resulting mixture was stirred for 2 hours at room temperature to obtain a yellow solution. The yellow solution was filtered and then freeze-dried. 3.472 Grams of sodium 4-[N-(3',4'-methylenedioxybenzylidene)aminomethyl]cyclohexane-1-carboxylate were obtained. The yield was 100%.

The characteristics of the sodium salt thus obtained were as follows:

(1) Melting point (according to capillary method):
270.0 to 273.0°C (decomposed)

(2) Elementary analytical data:

| | | | |
|---|---|---|---|
| Found (%) | C: 55.60, | H: 6.20, | N: 4.10 |
| Calcd.(%) as $C_{16}H_{18}NO_4.Na.2H_2O$ | C: 55.32, | H: 6.38, | N: 4.03 |

(3) Infrared absorption spectrum (KBr Tablet): as shown in Figure 1 of the accompanying drawings.

Example 3

Preparation of methyl 4-[N-(3',4'-methylenedioxybenzylidene)-aminomethyl]cyclohexane-1-carboxylate:

(a) After dropping 12.5 g of thionyl chloride into 82 ml of icewater-cooled methanol while stirring well, the cooling bath for methanol was removed, and then after adding 11.0 g of trans-4-aminomethylcyclohexane-1-carboxylic acid to the mixture, the thus formed mixture was heated for 5 hours at 50 to 60°C. Then, the reaction mixture was filtered while warm. The filtrate was concentrated to about 25 ml under a reduced pressure. The crystals educed on adding 140 ml of ether to the concentrated solution were collected by filtration after icewater-cooling and recrystallized from a mixture of methanol and ether to obtain 13.4 g of colourless plate-like crystals of methyl trans-4-aminomethylcyclohexane-1-carboxylate hydrochloride in a yield of 92.1% and giving the following elementary analytical data:

Elementary analytical data:

| | | | |
|---|---|---|---|
| Found (%) | C: 51.70, | H: 9.00, | N: 6.80 |
| Calcd.(%) as $C_9H_{18}NO_2Cl$ | C: 52.04, | H: 8.74, | N: 6.74 |

(b) After neutralizing 4.43 g of the thus obtained methyl trans-4-aminomethylcyclohexane-1-carboxylate hydrochloride with 20 ml of 1N sodium hydroxide solution, the solution was extracted three times with 30 ml portions of ethyl acetate. The extract after washing with water was dried with anhydrous sodium sulfate, and the solvent in the filtrate obtained by filtration of the dried extract was removed by evaporation under a reduced pressure in a warm water bath to obtain 2.70 g of a colourless liquid of methyl trans-4-aminomethylcyclohexane-1-carboxylate in a yield of 78.9% and giving the following elementary analytical data:

Elementary analytical data:

| | | | |
|---|---|---|---|
| Found (%) | C: 62.90, | H: 10.10, | N: 7.90 |
| Calcd.(%) as $C_9H_{17}NO_2$ | C: 63.12 | H: 10.01, | N: 8.18 |

(c) After dissolving 1.60 g (9.34 m mol) of the thus obtained methyl ester and 1.40 g (9.34 m mol) of piperonal into 40 ml of methanol, the solution was refluxed for 16 hours. Then, after removing the solvent from the solution under a reduced pressure in a warm water bath, the residue was dissolved in 40 ml of n-hexane. After filtering the solution while warm, the filtrate was subjected to recrystallization to obtain 2.68 g of colourless acicular crystals of methyl 4-[N-(3',4'-methylenedioxybenzylidene)-aminomethyl]cyclohexane-1-carboxylate in a yield of 94.7%, melting at 55.0 to 58.0°C (according to capillary method) and giving the following elementary analytical data and the infrared absorption spectrum as follows:

(1) Elementary analytical data:

| | | | |
|---|---|---|---|
| Found(%) | C: 67.20, | H: 7.10, | N: 4.60 |
| Calcd.(%) as $C_{17}H_{21}NO_4$ | C: 67.31, | H: 6.98, | N: 4.62 |

(2) Infrared absorption spectrum (KBr Tablet): as shown in Figure 2 of the accompanying drawings.

## Example 4

Preparation of ethyl 4-[N-(3′,4′-methylenedioxybenzylidene)-aminomethyl]cyclohexane-1-carboxylate

In a similar manner to Example 3, except for using ethanol instead of methanol, ethyl trans-4-aminomethylcyclohexane-1-carboxylate hydrochloride, ethyl trans-4-aminomethylcyclohexane-1-carboxylate and ethyl 4-[N-(3′,4′-methylenedioxybenzylidene)-aminomethyl]cyclohexane-1-carboxylate were synthesized in that order.

The respective physical properties of the products are as follows:

(a) Ethyl trans-4-aminomethylcyclohexane-1-carboxylate hydrochloride:
Yield: 14.2 g (91.6%)
Melting point (according to capillary method): 187~188°C
Elementary analytical data:

| | | | |
|---|---|---|---|
| Found(%) | C: 54.20, | H: 9.10, | N: 6.40 |
| Calcd.(%) as $C_{10}H_{20}NO_2Cl$ | C: 54.16; | H: 9.09; | N: 6.32 |

(b) Ethyl trans-4-aminomethylcyclohexane-1-carboxylate:
Yield: 3.10 g (83.8%)
Elementary analytical data:

| | | | |
|---|---|---|---|
| Found(%) | C: 64.80, | H: 10.00, | N: 7.80 |
| Calcd.(%) as $C_{10}H_{19}NO_2$ | C: 64.83, | H: 10.34; | N: 7.56 |

(c) Ethyl 4-[N-(3′,4′-methylenedioxybenzylidene)aminomethyl]-cyclohexane-1-carboxylate;
(1) Yield: 2.63 g (83.0%)
(2) Melting point: 72.5~73.5°C (according to capillary method)
(3) Elementary analytical data:

| | | | |
|---|---|---|---|
| Found(%) | C: 68.20, | H: 7.30, | N: 4.40 |
| Calcd.(%) as $C_{18}H_{23}NO_4$ | C: 68.12, | H: 7.30, | N: 4.41 |

(4) Infrared absorption spectrum (KBr Tablet): as shown in Figure 3 of the accompanying drawings.

## Example 5

Tests for inhibition of blood platelet aggregation and for acute toxicity of sodium 4-[N-3′,4′-methylene-dioxybenzylidene)-aminomethyl]cyclohexane-1-carboxylate:

5-1) Test for inhibition of platelet aggregation was carried out while using rabbit's platelets, an aqueous physiological saline solution of sodium 4-[N-(3′,4′-methylenedioxybenzylidene) aminomethyl]cyclohexane-1-carboxylate and 400 micromols of sodium arachidonate as the aggregation agent in a platelet aggregation tracer PAT 4A (made by Niko Bioscience Company, Japan) as the testing apparatus. The percent (%) inhibition of aggregation of the platelets by sodium arachidonate due to sodium 4-[N-(3′,4′-methylenedioxybenzylidene)aminomethyl]-cyclohexane-1-carboxylate at concentrations of 500, 1000 and 1500 micromols were found to be 30, 75 and 100, respectively.

5-2) Acute oral toxicity test was carried out by orally administering aqueous solutions of sodium 4-[N-(3′,4′-methylene-dioxybenzylidene)aminomethyl]cyclohexane-1-carboxylate to groups of female JCL-ICR mice five weeks after their birth. No abnormalities were found on the mice to which 3000 mg/kg of the test compound was administered.

## Example 6

Tests for inhibition of the migration of polymorphonuclear leukocytes to the inflammatory region:

Tests for the inhibition of the migration of polymorphonuclear leukocytes to the inflammatory region due to sodium 4-[N-(3′,4′-methylenedioxybenzylidene) aminomethyl]cyclohexane-1-carboxylate were carried out following the Granuloma-CMC-Pouch method (refer to Ishikawa et al. Yakugaku Zasshi, Vol. *88,* page 1472, 1968). Namely, after cutting the hair off a region of the back of male rats (Donryu, body weight: 150 g), 5 ml of air was injected subcutaneously in that region and, further, 5 ml of an aqueous 2% (W/V) solution of sodium carboxymethyl cellulose (CMC) at 37°C was injected. Sodium 4-[N-(3′,4′-methylenedioxybenzylidene)aminomethyl]cyclohexane-1-carboxylate was orally administered to one rat as a solution in aqueous physiological saline solution at a dose of 100 mg/kg at the time of injecting the solution of CMC, whilst 1 mg/kg of indomethacin was orally administered to another rat at the time of injecting the solution of CMC.

Specimens of the liquid (0.5 ml, each) which had effused into the pouch were collected 3, 6 and 24 hours after the 2% CMC solution had been injected. The number of polymorphonuclear leukocytes in each specimen was counted after staining to determine the rate of inhibition of migration of the polymorpholeukocytes as compared to the number of the polymorpholeukocytes in a specimen collected from a control animal to which only the aqueous CMC solution was injected after the air. The results are shown in the Table below.

TABLE

| Compound | Number of polymorphonuclear leukocytes at 3, 6 and 24 hours | | | | | |
|---|---|---|---|---|---|---|
| | 3 hours of injection of CMC | | 6 hours of injection of CMC | | 24 hours of injection of CMC | |
| | Number/$mm^3$ | R.I.(%)* | Number/$mm^3$ | R.I.(%) | Number/$mm^3$ | R.I.(%) |
| Present compound** | $1.9 \times 10^3$ | 46 | $2.7 \times 10^3$ | 54 | $10.1 \times 10^3$ | 32 |
| Indomethacin*** | $2.3 \times 10^3$ | 34 | $4.1 \times 10^3$ | 31 | $10.8 \times 10^3$ | 28 |
| Control | $3.5 \times 10^3$ | – | $5.9 \times 10^3$ | – | $14.9 \times 10^3$ | – |

Notes: R.I.(%)* means the inhibitory rate of migration of polymorphonuclear leukocytes into the pouch as compared to control.

Present compound**: Sodium 4–[N–(3′,4′-methylenedioxybenzylidene)aminomethyl]cyclohexane-1-carboxylate orally administered once at 100 mg/kg, at the time of injecting sodium CMC.

Indomethacin***: Orally administered once at 10 mg/kg, at the time of injecting sodium CMC.

## Claims

1. A compound represented by the formula (I)

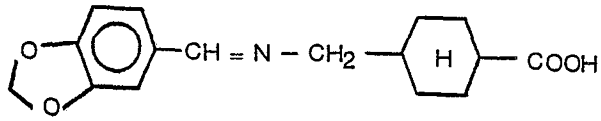

(I)

or a $C_1$—$C_4$ alkyl ester thereof or a pharmaceutically acceptable salt thereof.

2. A compound according to claim 1, which is 4-[N-(3',4'-methylenedioxybenzylidene)aminomethyl]cyclohexane-1-carboxylic acid.

3. A compound according to claim 1, which is methyl 4-[N-(3',4'-methylenedioxybenzylidene)-aminomethyl]cyclohexane-1-carboxylate.

4. A compound according to claim 1, which is ethyl 4-[N-(3',4'-methylenedioxybenzylidene)-aminomethyl]cyclohexane-1-carboxylate.

5. A compound according to claim 1, which is sodium 4-[N-(3',4'-methylenedioxybenzylidene)-aminomethyl]cyclohexane-1-carboxylate.

6. A compound according to any one of the preceding claims for use in the treatment of inflammation, thrombosis, hypertension, asthma, cancer, rheumatism or systemic lupus erthematosus.

7. A process for preparing a compound of formula (I) or an ester or salt thereof as claimed in claim 1, which process comprises reacting 3,4-methylenedioxybenzaldehyde with 4-aminomethylcyclohexane-1-carboxylic acid or a $C_1$—$C_4$ alkyl ester thereof, and optionally converting the resulting compound into a pharmaceutically acceptable salt of the compound of formula (I).

8. A pharmaceutical composition which comprises as active ingredient a compound of formula (I) as defined in claim 1 or a $C_1$—$C_4$ alkyl ester thereof or a pharmaceutically acceptable salt thereof, together with a pharmaceutically acceptable carrier or diluent.

9. A pharmaceutical composition in unit dosage form, which composition comprises a therapeutically effective amount of a compound of formula (I) as defined in claim 1 or a $C_1$—$C_4$ alkyl ester thereof or a pharmaceutically acceptable salt thereof, together with a pharmaceutically acceptable carrier or diluent.

## Patentansprüche

1. Verbindung, gekennzeichnet durch die nachstehende Formel (I)

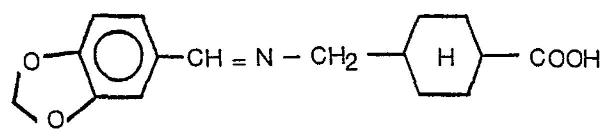

(I)

oder ein $C_1$—$C_4$ Alkylester dieser Verbindung, oder ein pharmazeutisch akzeptables Salz dieser Verbindung.

2. Verbindung nach Anspruch 1, dadurch gekennzeichnet, daß sie die 4-[N-(3',4'-Methylendioxybenzyliden)aminomethyl]cyclohexan-1-carbonsäure ist.

3. Verbindung nach Anspruch 1, dadurch gekennzeichnet, daß sie das Methyl-4-[N-(3',4'-Methylendioxybenzyliden)aminomethyl]cyclohexan-1-carboxylat ist.

4. Verbindung nach Anspruch 1, dadurch gekennzeichnet, daß sie das Ethyl-4-[N-(3',4'-Methylendioxybenzyliden)aminomethyl]cyclohexan-1-carboxylat ist.

5. Verbindung nach Anspruch 1, dadurch gekennzeichnet, daß sie das Natrium-4-[N-(3',4'-Methylendioxybenzyliden)aminomethyl]cyclohexan-1-carboxylat ist.

6. Verbindung nach einem der Ansprüche 1 bis 5 als Mittel zur Behandlung von Entzündungen, Thrombose, Hypertonie, Asthma, krebs, Rheumatismus oder Lupus erythematodes.

7. Verfahren zur Herstellung einer Verbindung mit der Formel (I) oder eines Esters dieser Verbindung gemäß Anspruch 1, dadurch gekennzeichnet, daß man 3,4-Methylendioxybenzaldehyd mit 4-Aminomethylcyclohexan-1-carbonsäure oder einem $C_1$—$C_4$-Alkylester dieser Säure umsetzt und gegebenenfalls die gebildete Verbindung in ein pharmazeutisch akzeptables Salz der Verbindung mit der Formel (I) umwandelt.

8. Pharmazeutisches Präparat, dadurch gekennzeichnet, daß es als wirksamen, Bestandteil eine Verbindung mit der Formel (I) gemäß Anspruch 1, oder einen $C_1$—$C_4$-Alkylester dieser Verbindung, oder ein pharmazeutisch akzeptables Salz dieser Verbindung, zusammen mit einem pharmazeutisch akzeptablen Träger oder Verdünnungsmittel enthält.

9. Pharmazeutisches Präparat in einheitlicher Dosisform, dadurch gekennzeichnet, daß es eine pharmazeutisch wirksame Menge einer Verbindung mit der Formel (I) gemäß Anspruch 1 oder einen $C_1$—$C_4$-Alkylester oder ein pharmazeutisch akzeptables Salz dieser Verbindung zusammen mit einem pharmazeutisch akzeptablen Träger oder Verdünnungsmittel enthält.

**Revendications**

1. Un composé représenté par la formule (I)

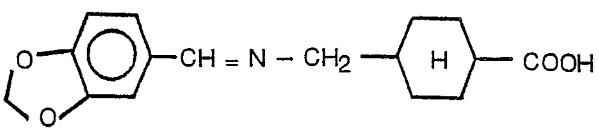

(I)

ou par un ester alkyl $C_1$—$C_4$ dérivé de cette formule, ou par un sel pharmaceutiquement acceptable de celui-ci.

2. Un composé selon la revendication 1, qui est un acide cyclohexane-1-carboxylique 4-[N-3′,4′-méthylène-dioxybenzylidène)aminométhyle].

3. Un composé selon la revendication 1, qui est un carboxylate-1-cyclohexane de méthyle 4-[N-(3′,4′-méthylène-dioxybenzylidène)aminométhyle].

4. Un composé selon la revendication 1, qui est un carboxylate-1-cyclohexane d'éthyle 4-[N-(3′,4′-méthylène-dioxybenzylidène)aminométhyle].

5. Un composé selon la revendication 1, qui est un carboxylate-1-cyclohexane de sodium 4-[N-(3′,4′-méthylène-dioxybenzylidène)aminométhyle].

6. Un composé selon une quelconque des revendications précédentes, destiné à être utilisé dans le traitement d'inflammations, de thrombose, d'hypertension, d'asthme, de cancer, de rhumatisme, ou d'erthématose lupus systémique.

7. Une méthode pour la préparation d'un composé de formule (I) ou d'un ester ou sel à partir de cette formule, selon ce qui est revendiqué dans la revendication 1, ladite méthode comprenant la mise en réaction de méthylène-dioxybenzaldéhyde-3,4 avec de l'acide 4-aminométhylcyclohexane-1-carboxylique, ou avec un ester alkyl $C_1$—$C_4$ de celui-ci, et la conversion facultative du composé obtenu en un sel pharmaceutiquement acceptable du composé de la formule (1).

8. Une composition pharmaceutique qui comprend comme ingrédient actif un composé de la formule (I) tel que défini dans la revendication 1, ou un ester alkyl $C_1$—$C_4$ dérivé de cette formule, ou un sel pharmaceutiquement acceptable de ce composé, conjointement avec un excipient ou un diluant pharmaceutiquement acceptable.

9. Une composition pharmaceutique sous forme de dosage unitaire, ladite composition comprenant une quantité thérapeutiquement efficace d'un composé de la formule (I) comme défini dans la revendication 1, ou d'un ester alkyl $C_1$—$C_4$ dérivé de cette formule, ou d'un sel pharmaceutiquement acceptable de ce composé, conjointement avec un excipient ou un diluant pharmaceutiquement acceptable.

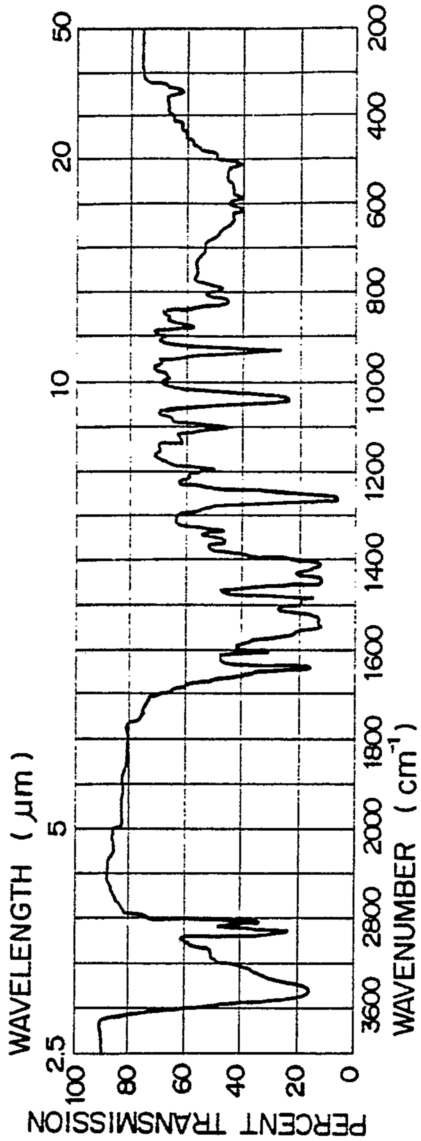
WAVELENGTH ( μm )
2.5
5
10
20
50
PERCENT TRANSMISSION
100
80
60
40
20
0
WAVENUMBER ( cm-1 )
3600
2800
2000
1800
1600
1400
1200
1000
800
600
400
200

Fig 1

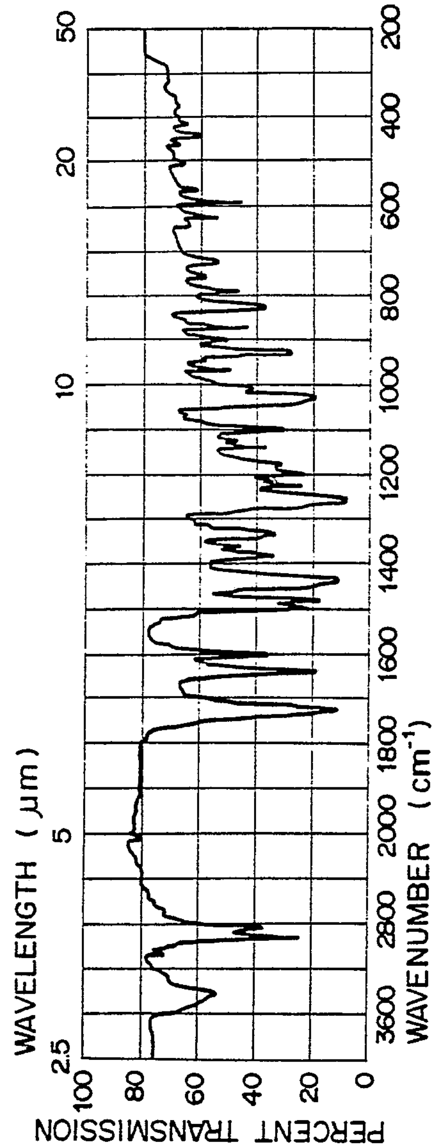
WAVELENGTH ( μm )
2.5
5
10
20
50
PERCENT TRANSMISSION
100
80
60
40
20
0
WAVENUMBER ( cm-1 )
3600
2800
2000
1800
1600
1400
1200
1000
800
600
400
200

Fig 2

Fig 3

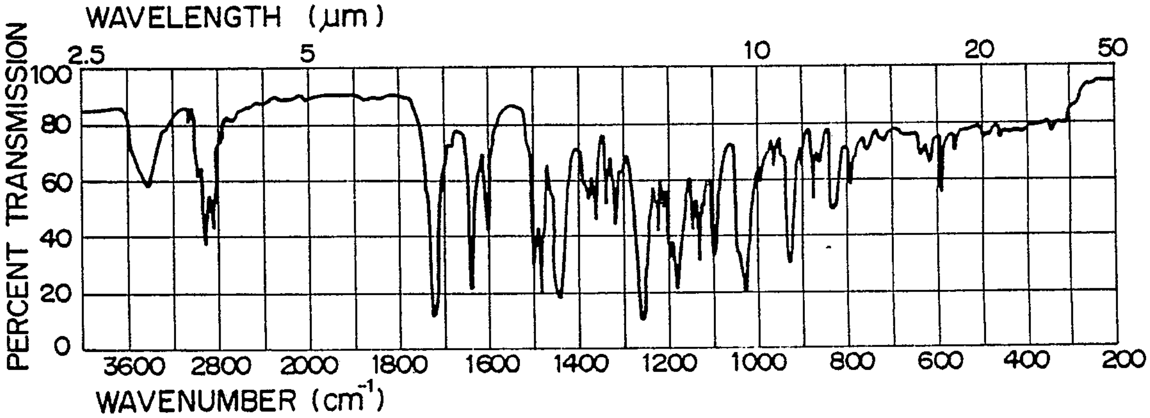
WAVELENGTH (μm)
2.5
5
10
20
50
PERCENT TRANSMISSION
100
80
60
40
20
0
3600
2800
2000
1800
1600
1400
1200
1000
800
600
400
200
WAVENUMBER (cm⁻¹)